(19) Europäisches Patentamt
European Patent Office
Office européen des brevets

(11) **EP 1 366 038 B1**

(12) **FASCICULE DE BREVET EUROPEEN**

(45) Date de publication et mention de la délivrance du brevet:
**31.08.2005 Bulletin 2005/35**

(21) Numéro de dépôt: **02704891.7**

(22) Date de dépôt: **25.02.2002**

(51) Int Cl.[7]: **C07D 401/14**, A61K 31/404, A61P 43/00, C07D 401/06, C07D 409/14

(86) Numéro de dépôt international:
**PCT/FR2002/000681**

(87) Numéro de publication internationale:
**WO 2002/068411 (06.09.2002 Gazette 2002/36)**

(54) **OXINDOLES INHIBITEURS DE CDK-1 ET LEUR APPLICATION EN THERAPEUTIQUE**
OXINDOLDERIVATE ALS CDK-1 INHIBITOREN UND DEREN VERWENDUNG IN THERAPIE
CDK-1 INHIBITOR OXINDOLES AND THE APPLICATION THEREOF IN THERAPEUTICS

(84) Etats contractants désignés:
**AT BE CH CY DE DK ES FI FR GB GR IE IT LI LU MC NL PT SE TR**

(30) Priorité: **27.02.2001 FR 0102624**

(43) Date de publication de la demande:
**03.12.2003 Bulletin 2003/49**

(73) Titulaire: **Aventis Pharma S.A.**
**92160 Antony (FR)**

(72) Inventeurs:
- **RIOU, Jean-François**
  **F-51100 Reims (FR)**
- **MARATRAT, Michel**
  **F-94290 Villeneuve la Roi (FR)**
- **GRONDARD, Lucile**
  **F-91080 Courcouronnes (FR)**
- **THOMPSON, Fabienne**
  **F-75012 Paris (FR)**
- **PETITGENET, Odile**
  **F-75014 Paris (FR)**
- **MAILLIET, Patrick**
  **F-94120 Fontenay sous Bois (FR)**
- **LAVAYRE, Jacques**
  **F-94440 Marolles en Brie (FR)**

(56) Documents cités:
**WO-A-96/40116**
**WO-A-98/07695**
**WO-A-98/50356**
**WO-A-99/62503**
**US-B1- 6 313 310**

- **LI SUN ET AL.: "Synthesis and biological evaluation of 3-substituted indolin-2-ones: a novel class of tyrosine kinase inhibitors that exhibit selectivity toward particular receptor tyrosine kinases" JOURNAL OF MEDICINAL AND PHARMACEUTICAL CHEMISTRY., vol. 41, no. 14, - 1998 pages 2588-2603, XP002184621 AMERICAN CHEMICAL SOCIETY. EASTON., US**
- **CHEMICAL ABSTRACTS, vol. 87, no. 19, 7 novembre 1977 (1977-11-07) Columbus, Ohio, US; abstract no. 151939k, KOVAC,J. ET AL.: "Furan derivatives. LXXX. Synthesis and properties of substituted furfurylideneoxindoles" XP002184622 & CHEM. ZVESTI, vol. 30, no. 4, - 1976 pages 484-492, -& DATABASE CHEMICAL ABSTRACTS [en ligne] CA 87:151939, XP002184623**

EP 1 366 038 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne une famille d'oxindoles qui sont des inhibiteurs chimiques du complexe enzymatique Cdc2/cycline B (CDK-1) et leur application en thérapeutique.

Comme cela est bien connu, le cycle cellulaire d'un eucaryote comporte différentes étapes (voir figure 1) :

Après la phase M, qui se compose d'une division nucléaire (mitose) et d'une division cytoplasmique (cytodiérèse), les cellules filles commencent l'interphase d'un nouveau cycle. Cette interphase commence avec la phase G1 pendant laquelle on note une reprise accrue des activités de biosynthèse de la cellule. La phase S commence quand la synthèse de l'ADN démarre et se termine quand les chromosomes se sont répliqués (chaque chromosome est alors composé de deux chromatides soeurs identiques). La cellule entre ensuite en phase G2 (dernière phase de l'interphase) qui se poursuit jusqu'au début de la mitose, initiant la phase M. Au cours de cette phase, les chromatides soeurs se séparent, deux nouveaux noyaux se forment et le cytoplasme se divise pour donner deux cellules filles dotées chacune d'un noyau. La cytodiérèse termine la phase M et marque le début de l'interphase du cycle cellulaire suivant.

La machinerie moléculaire du cycle cellulaire est composée de facteurs de régulation qui contrôlent la progression dans le cycle cellulaire. Le passage d'une phase à l'autre dans un cycle est sous le contrôle d'une famille de protéines sérine/thréonine kinases de petite taille, les kinases dépendantes des cyclines (CDK) qui régulent l'activité de protéines par phosphorylation. Le taux d'expression des CDK est à peu près constant au cours du cycle cellulaire, mais ces protéines kinases sont inactives en elles-mêmes et doivent être activées pour acquérir une activité kinasique. L'activité enzymatique et la spécificité des CDK dépendent de leur association avec une sous-unité régulatrice appartenant à la famille des cyclines.

L'entrée en mitose, en particulier, est sous le contrôle d'un facteur promoteur de la phase M (ou MPF) qui est constitué par l'assemblage d'une molécule de (CDK1=p34cdc2) et d'une molécule de cycline B. Le complexe cycline B-CDK1 activé permet la transition G2/M en phosphorylant de nombreux substrats.

Ainsi, la modulation de l'activité du complexe cycline B-CDK1 est un mécanisme clé de l'arrêt de la prolifération cellulaire et les CDK constituent des cibles moléculaires privilégiées dans la recherche d'inhibiteurs sélectifs de la prolifération cellulaire. En effet, certaines propriétés des CDK (en particulier les altérations très fréquentes, dans les tumeurs humaines, des CDK et de leurs régulateurs) et de leurs inhibiteurs protéiques naturels ont encouragé la recherche d'inhibiteurs chimiques de CDK en vue d'applications antitumorales.

De nombreux composés ont ainsi été testés et reconnus comme inhibiteurs de CDK : les purines, les paullones, le flavopiridol, les indirubines, l'olomoucine, la roscovitine, la butyrolactone-1, la toyocamycine et d'autres. Les inhibiteurs chimiques qui agissent plus particulièrement sur le complexe cycline B/CDK1 empêchent en fait la phosphorylation de substrats tels que l'histone H1, les sous-unités γ et δ du facteur d'élongation ou la vimentine.

Ainsi, les premiers inhibiteurs chimiques de CDK présentent des propriétés intéressantes qui justifient leur évaluation comme produits anticancéreux potentiels et la poursuite de la recherche de nouvelles molécules plus efficaces.

La demande de brevet WO 96/40116 décrit des oxindoles utilisés dans le but de moduler le signal de transduction de la protéine tyrosine kinase (PTK).

La demanderesse a découvert une famille de composés de la famille des oxindoles ayant une action marquée sur la prolifération cellulaire et sur CDK-1.

L'invention a pour objet des composés nouveaux de la famille des oxindoles.

Un autre objet est constitué par leur application comme médicament.

D'autres objets apparaîtront à la lumière de la description et des exemples qui suivent.

La présente invention a ainsi pour objet les composés répondant à la formule (I) :

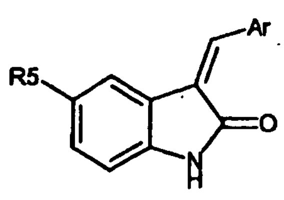

dans laquelle R5 est choisi parmi les groupements 3-pyridinyle, 5-pyrimidinyle, -CONH-alkyle en C1-C4, -NHCO-alkyle en C1-C4, CO2R où R peut être hydrogène ou alkyle en C1-C4, -SO2NH2, -CF3,

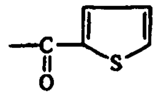

dans laquelle Ar est choisi parmi les groupements 5-imidazolyle, 2-pyrrolyle éventuellement substitués par un radical alkyle en C1-C4, 2-furyle, ou 2-thiazolyle, sous la forme E, Z ou un mélange des deux formes d'isomères.

Dans les composés de formule (I), R5 désigne préférentiellement un 5 groupement 3-pyridinyle ou -CONH-méthyle ou -NHCO-méthyle et Ar désigne préférentiellement un groupement 5-imidazolyle ou un 5-(4-methyl-imidazolyle).

La présente invention a tout particulièrement pour objet les composés de formule (I) telle que définie ci-dessus, répondant aux formules suivantes :

3-(imidazol-4-méthylidènyl-5-(pyridin-3-yl)-indolin-2-one,
3-(pyrrol-2-méthylidènyl)-5-(pyridin-3-yl)-indolin-2-one,
3-(imidazol-4-méthylidènyl)-5-N-méthylcarboxamido-indolin-2-one,
3-(imidazol-4-méthylidènyl)-5-acétylamino-indolin-2-one.

Les composés de formule générale (I), dans lesquels R5 et Ar ont les significations précitées, peuvent être obtenus par couplage d'une indolin-2-one de 15 formule générale (II), dans laquelle R5 a la signification précitée, avec un aldéhyde aromatique de formule générale (III), dans lequel Ar a la signification précitée, selon le schéma ci-dessous :

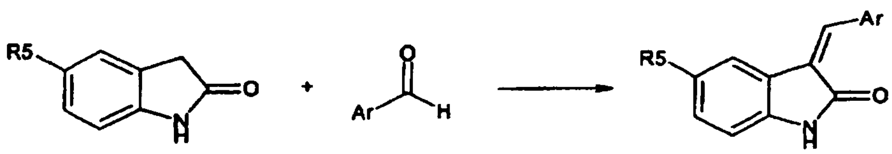

(II) (III) (I)

La réaction de couplage s'effectue généralement dans les conditions décrites par E. Knoevenagel (Chem. Ber. 1900, 23, 172), à savoir dans un solvant protique comme le methanol où l'éthanol, en présence d'une quantité catalytique de base organique telle que la pipéridine, à une température comprise entre 20°C et la température de reflux du solvant utilisé.

Les indolin-2-ones de formule générale (II) et les aldéhydes aromatiques de formule (III), dans lesquels respectivement R5 et Ar ont les significations précitées, sont soit commerciaux soit préparés selon les conditions décrites dans la littérature.

Des exemples de préparation sont décrits ci-après.

La demanderesse a découvert que les composés de formule (I) conformes à l'invention possèdent des propriétés inhibitrices de protéines kinases (CDK). Ces protéines jouent un rôle clé dans l'initiation, le développement et l'achèvement des événements du cycle cellulaire. Ainsi, les molécules inhibitrices de CDK sont susceptibles de limiter les proliférations cellulaires inopportunes telles que celles observées dans les cancers.

La protéine kinase CDK1 est particulièrement sensible aux effets inhibiteurs des composés de la présente invention.

Les produits de la présente invention possèdent en plus de leurs propriétés inhibitrices spécifiques de la protéine kinase CDK-1, des effets cellulaires tels que des propriétés antiprolifératives par blocage de la division cellulaire en cours de cycle et apoptotiques par induction de l'apoptose cellulaire.

Les composés conformes à l'invention sont notamment utiles dans le cadre de thérapie de tumeurs primaires et de localisations secondaires des cancers.

L'invention a donc pour objet leur utilisation comme médicaments pouvant être utilisés seuls ou en combinaison avec des traitements tels que la chimiothérapie, la radiothérapie ou les traitements anti-angiogéniques mettant éventuellement en oeuvre d'autres substances actives. L'invention s'étend aux compositions pharmaceutiques contenant à titre de principe actif l'un au moins des composés de la formule (I) telle que définie ci-dessus dans un milieu pharmaceutiquement acceptable.

Ces compositions pharmaceutiques peuvent être administrées par voie buccale, par voie parentérale ou par voie locale en application topique sur la peau et les muqueuses ou par injection par voie intraveineuse ou intramusculaire. Ces compositions peuvent être solides ou liquides et se présenter sous toutes les formes pharmaceutiques couramment utilisées en médecine humaine comme, par exemple, les comprimés simples ou dragéifiés, les pilules,

les tablettes, les gélules, les gouttes, les granulés, les préparations injectables, les pommades, les crèmes ou les gels. Elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants, ou émulsifiants, les conservateurs.

La posologie usuelle, variable selon le produit utilisé et le sujet traité peut être, par exemple, de 0,05 à 5 grammes par jour chez l'adulte.

Les exemples suivants illustrent l'invention sans toutefois la limiter :

**<u>Exemple 1</u> : 3-(imidazol-4-méthylidènyl-5-(pyridin-3-yl)-indolin-2-one**

A une solution de 0,61 g (3 mmoles) de 5-(pyridin-3-yl)-indolin-2-one dans 75 mL d'éthanol contenant 0,02 mL de pipéridine, on ajoute 0,28 g (3 mmoles) d'imidazole-4-carboxaldéhyde. Le milieu réactionnel est porté au reflux pendant 4 heures. Après refroidissement, le précipité formé est essoré, lavé par 2 fois 5 mL d'éthanol glacé et séché sous pression réduite. On obtient ainsi 0,62 g (75 %) de 3-(imidazol-4-méthylidènyl)-5-(pyridin-3-yl)-indolin-2-one, sous forme d'un solide jaune citron dont la caractéristique est la suivante : point de fusion = 310°C.

**<u>Exemple 2</u> : 3-(pyrrol-2-méthylidènyl)-5-(pyridin-3-yl)-indolin-2-one**

En opérant comme à l'exemple 1, mais à partir de 2,1 g (10 mmoles) de 5-(pyridin-3-yl)-indolin-2-one dans 150 mL d'éthanol et de 0,99 g (10 mmoles) de pyrrole-2-carboxaldéhyde, on obtient 2,66 g (92,5 %) de 3-(pyrrol-2-méthylidènyl)-5-(pyridin-3-yl)-indolin-2-one, sous forme d'un solide orange dont la caractéristique est la suivante : point de fusion = 225°C.

**<u>Exemple 3</u> : 3-(imidazol-4-méthylidènyl)-5-N-méthylcarboxamido-indolin-2-one**

En opérant comme à l'exemple 1, mais à partir de 0,2 g (1,05 mmoles) de 5-méthylcarboxamido-indolin-2-one dans 25 mL d'éthanol et de 0,1 g (1,04 mmoles) de imidazole-5-carboxaldéhyde, on obtient 0,21 g (84 %) de 3-(imidazol-4-méthylidènyl-5-N-méthylcarboxamido-indolin-2-one, sous forme d'un solide orange dont la caractéristique est la suivante : point de fusion > 260°C.

**<u>Exemple 4</u> : 3-(imidazol-4-méthylidènyl)-5-acétylamino-indolin-2-one**

En opérant comme à l'exemple 1, mais à partir de 0,4 g (2,1 mmoles) de 5-acétylamino-indolin-2-one dans 50 mL d'éthanol et de 0,2 g (2 mmoles) de imidazole-4-carboxaldéhyde, on obtient 0,31 g (58 %) de 3-(imidazol-4-méthylidènyl)-5-acétylamino-indolin-2-one, sous forme d'un solide orange dont la caractéristique est la suivante : point de fusion > 260°C.

**<u>Exemple 5</u> : synthèse en parallèle de 3-(aryl)méthylydènyl-indolin-2-one de formule générale (I)**

Dans un réacteur magnétique chauffant avec condenseur Zymark, de type STEM RS2050 contenant 25 puits en parallèle munis chacun d'un tube en verre de 50 mL, on introduit 0,5 mmole d'indolyl-2-one de formule générale (II), 0,5 mmole d'un aldéhyde aromatique de formule générale (III), 5 mL d'éthanol et 1 goutte de pipéridine. Le milieu réactionnel est porté au reflux pendant une nuit. Après refroidissement, et dilution avec 5 mL d'eau, le précipité formé est essoré et séché sous pression réduite. On obtient ainsi les 3-(aryl)méthylydènyl-indolin-2-ones de formule générale (I), représentées par exemple et à titre non limitatif par les composés 5-1 à 5-14.

**<u>Exemple 6</u> : Dosage de l'inhibition de l'activité p34cdc2/cycline B par les composés selon l'invention :**

L'inhibition de l'activité p34cdc2/cycline B (CDK1/cycline B) est déterminée par un protocole qui permet de mesurer l'activité de transfert par l'enzyme, d'un groupement $^{32}P$, à partir d'ATP[$\gamma^{32}P$] sur un substrat, l'histone H1.

$$\text{Histone H1} + {}^{32}\text{P-ADP} \xrightarrow{\text{CDK-1} + \text{Composé à tester}} {}^{32}\text{P-Histone H1} + \text{ADP}$$

Les préparations d'enzymes utilisées correspondent soit à l'enzyme p34$^{cdc2}$/cycline B d'étoile de mer (fournie par L. Meijer, CNRS, Station Biologique, Roscoff, France), soit à l'enzyme p34$^{cdc2}$/cycline B humaine recombinante (fournie par New England Biolabs, Inc., Beverly, MA01915, USA). La protéine Histone H1 (type III-S) est obtenue auprès de Sigma.

Le tampon C contient 60 mM de β-glycérophosphate, 30 mM de nitro-phenylphosphate, 25 mM de MOPS pH 7.0, 5 mM d'EGTA, 15 mM de MgCl2, 1 mM de dithiothreitol et 0.1 mM d'orthovanadate. La solution d'ATP est préparée en mélangeant 20 µL d'ATP [$\gamma^{32}$P] à (3000 Ci/mmole) et 90 µL d'ATP non radioactif à 1 mM dans 890 µL de tampon C.

Le milieu réactionnel est préparé selon la composition suivante :

10 µL de préparation enzymatique sont ajoutés à 5 µL d'Histone H1 (5 mg/ml dans du tampon C) et à 7 µL de tampon C et mélangés. 17,5 µL du mélange sont répartis dans les tubes juste avant l'essai. 3 µL d'agent inhibiteur à tester sont ajoutés dans chaque tube.

La réaction est démarrée par l'addition de 5 µL de la solution d'ATP suivie d'une incubation de 15 minutes à 30°C. La réaction est arrêtée par l'addition de 0.5 volumes de tampon Laemli 3X. Après chauffage de l'échantillon pendant 5 minutes à 90°C, les échantillons sont ensuite analysés par électrophorèse en gel de protéine à 10 % d'acrylamide pendant 1 heure sous une tension de 200 volts à l'aide d'un système d'électrophorèse Novex. Les gels d'acrylamide sont ensuite séchés sur une feuille de papier whatmann 3MM à 80°C pendant 1 heure.

L'analyse et la quantification de la phosphorylation de l'Histone H1 sont effectués à l'aide d'un appareil Instant-Imager (Packard). Pour chaque concentration de composé testée, les résultats sont exprimés en pourcentage d'inhibition de la réaction et calculés à partir du contrôle enzymatique non traité.

La concentration de composé induisant une inhibition de 50 % de la réaction de phosphorylation de p34$^{cdc2}$/cycline B (IC50) est déterminée à l'aide d'une représentation graphique semi-logarithmique des valeurs d'inhibition obtenues en fonction de chacune des concentrations de composé testée.

<u>Inhibition de CDK1 par les composés de l'invention selon la formule (I)</u>

| N° | R5 | Ar | CDK1 % inhibition à 10 µM | CDK1 IC50 en nM |
|---|---|---|---|---|
| 1 | 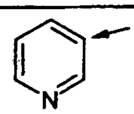 | 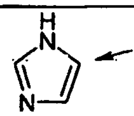 | 99 | 0,3 |
| 2 | 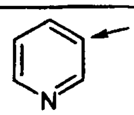 | 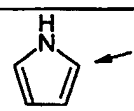 | 87 | 2 |
| 3 | 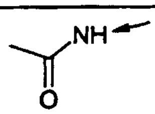 | 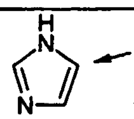 | 100 | 2.5 |
| 4 | 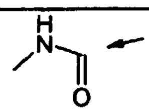 | 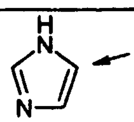 | 100 | 0,7 |
| 5-1 | 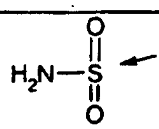 | 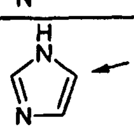 | 86 | |

| | | | | |
|---|---|---|---|---|
| 5-2 | 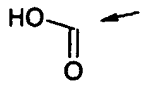 | 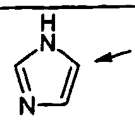 | 85 | |
| 5-3 | Br | 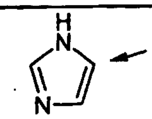 | 100 | 16 |
| 5-4 | 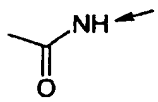 | 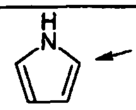 | 80 | |
| 5-5 | 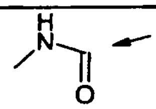 | 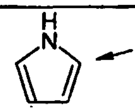 | 99 | |
| 5-6 | 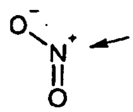 | 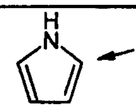 | 91 | 21 |
| 5-7 | 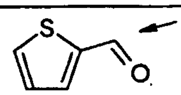 | 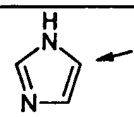 | 100 | 10 |
| 5-8 | 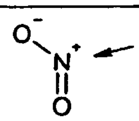 | 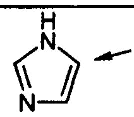 | 81 | 10 |
| 5-9 | 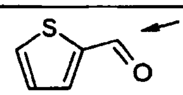 | 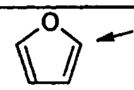 | 75 | |
| 5-10 | 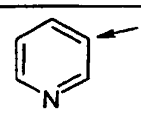 | 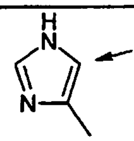 | 100 | 16 |
| 5-11 | 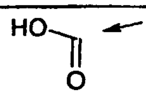 | 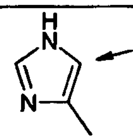 | 100 | 6 |
| 5-12 | 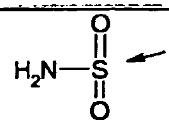 | 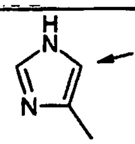 | 100 | 3,5 |
| 5-13 | 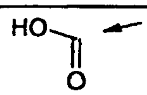 | 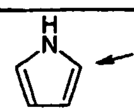 | 79 | |
| 5-14 | 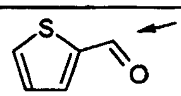 | 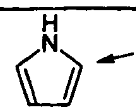 | 62 | |

On considère qu'un composé est actif en tant qu'agent anti-P34$^{cdc2}$/cycline B lorsque la CI50 est inférieure à 5 µM (5000 nM d'après les unités de mesure utilisées dans le tableau). Plusieurs composés peuvent donc être considérés comme inhibiteurs du complexe CDK1/cycline B et en particulier le n°1.

**Exemple 7 : Détermination de l'inhibition de clonogénicité :**

Les lignées de cellules humaines KB, HCT-116, HT-29, HCT-8, Lovo, PC-3, PC-14, HLF et HLE ainsi que la lignée de tumeur de rat C6 proviennent de l'ATCC (American Type Culture Collection, Rockville USA). La lignée de tumeur humaine Calc18 est un don du Professeur G. Riou (Institut Gustave Roussy, Villejuif, France). Les cellules HCT-8, Lovo, PC-3, PC-14, HLF et HLE sont cultivées en couche en flacons de culture dans du milieu RPMI 1640, L-Glutamine à 2 mM, Pénicilline 200 U/ml streptomycine 200 µg/mL et additionné de 10 % de sérum foetal de veau inactivé par la chaleur. Les cellules HCT-116, HT-29, KB, C6 et Calcl8 sont cultivées en couche en flacon de culture dans du milieu de Dulbeco's contenant L-Glutamine à 2 mM, Pénicilline 200 U/mL streptomycine 200 µg/mL et additionné de 10 % de sérum foetal de veau inactivé par la chaleur.

Les cellules en phase exponentielle de croissance sont trypsinées, lavées dans du PBS 1X et sont diluées à une concentration finale de 5000 cellules/mL dans du milieu complet. Les produits à tester (dans un volume de 50 µL) sont ajoutés à 2,5 mL de suspension. Ensuite, 0.4 mL d'Agar Noble Difco à 2.4 % maintenu à une température de 45°C sont ajoutés aux cellules. Le mélange est ensuite immédiatement versé dans des boites de Pétri et laissé à +4°C pendant 5 minutes pour solidifier la gélose. Le nombre de clones cellulaires (>60 cellules) est mesuré après 12 jours d'incubation à 37°C sous atmosphère de 5 % de CO2.

Le composé n°1 est testé à des concentrations de 10, 1, 0.1, 0.01 µM en duplicats. Les résultats sont exprimés en pourcentage d'inhibition de la clonogénicité par rapport aux contrôles. La CI50 est déterminée graphiquement à partir des résultats moyens déterminés pour chaque concentration de composé.

| Inhibition de la clonogénicité en agar par le composé n°1 sur des lignées cellulaires tumorales | | |
|---|---|---|
| Lignée cellulaire | Tissu d'origine | CI50 (µM) |
| HCT-116 | colon | 0.28 |
| HT-29 | colon | 1.42 |
| HCT-8 | colon | 1.04 |
| Lovo | colon | 0.9 |
| Calc18 | sein | 1.06 |
| PC-3 | prostate | 0.19 |
| PC-14 | poumon | 1.1 |
| HLF | foie | 1.1 |
| HLE | foie | 1.2 |
| C6 | glioblastome | 10.8 |

On considère qu'un composé est actif comme agent cytotoxique si la CI50 est inférieure à 10 µM ce qui est le cas pour toutes les lignées cellulaires testées avec le composé n°1 (à l'exception du glioblastome de rat C6).

**Exemple 8 : Mesure de l'inhibition de prolifération par les composés n°1 ou n°2.**

Les cellules HCT-116 sont cultivées en couche en flacon de culture dans du milieu de Dulbeco's contenant L-Glutamine à 2 mM, Pénicilline 200 U/mL streptomycine 200 µg/mL et additionné de 10 % de sérum foetal de veau inactivé par la chaleur.

Les cellules HCT-116 en phase exponentielle de croissance sont trypsinées, lavées dans du PBS 1X et sont ensemencées en microplaques 96 puits (Costar) à raison de 4x104 cellules/mL et de 1,5x104 cellules/mL (0.2 mL/puit) puis incubées pendant 96 heures en présence de concentrations variables de produit à étudier (10, 1, 0.1 et 0.01 µg/mL, chaque point en quadruplicat). Seize heures avant la fin de l'incubation, 0.02 % final de rouge neutre est ajouté dans chaque puits. A la fin de l'incubation, les cellules sont lavées par du PBS 1X et lysées par 1 % de lauryl sulfate de sodium. L'incorporation cellulaire du colorant, qui reflète la croissance cellulaire, est évaluée par spectrophotométrie à une longueur d'onde de 540 nm pour chaque échantillon à l'aide d'un appareil de lecture Dynatech MR5000.

Pour chaque concentration de composé testée, les résultats sont exprimés en pourcentage d'inhibition de croissance cellulaire et calculés à partir du contrôle non traité et du milieu de culture sans cellules (blanc) selon la formule suivante :

(Valeur Composé - valeur blanc / Valeur contrôle cellules -valeur blanc)x 100.

La concentration de composé induisant une inhibition de 50 % de la croissance (IC50) est déterminée à l'aide d'une représentation graphique semi-logarithmique des valeurs d'inhibition obtenues en fonction de chacune des concentrations de composé testées.

**Inhibition de la prolifération des cellules HCT-116 par les composés 1 ou 2**

| N° | R5 | Ar | HCT-116 CI50 µM |
|---|---|---|---|
| 1 | 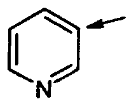 | 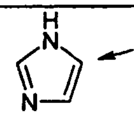 | 0.28 |
| 2 | 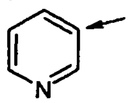 | 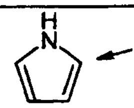 | 8.0 |

On considère qu'un composé est actif comme agent cytotoxique si dans l'une ou l'autre des méthodes, la CI50 est inférieure à 10 µM ce qui est le cas pour les deux composés testés dans cette expérience.

**Exemple 9 : Détermination de l'activité pro-apoptotique et de blocage mitotique sous l'action du composé n°1 :**

Après exposition pendant 48 heures de cellules HCT-8 au composé n°1, les cellules en culture sont trypsinées, lavées au PBS 1X et déposées entre lame et lamelle en présence de Hoechst 33342 à une concentration de 1 µg/mL. Le pourcentage de cellules mitotiques et de cellules possédant des corps nucléaires apoptotiques est déterminé par examen et comptage d'un échantillon d'au moins 300 cellules réparties en plusieurs endroits de la lame à l'aide d'un microscope à fluorescence.

| Induction d'apoptose et blocage mitotique induits par le composé n°1 sur la lignée HCT-8 | | | | |
|---|---|---|---|---|
| Concentration testée du composé n°1 | Cellules apoptotiques (%) | Facteur/ témoin | Cellules mitotiques (%) | Facteur/ témoin |
| 0 µg/mL (Contrôle) | 2.4 | 1 | 6,9 | 1 |
| 0.1 µg/mL | 8.5 | 3,5 | 2,8 | 0,4 |
| 1 µg/mL | 16 | 6,6 | 0,7 | 0,1 |
| 10 µg/mL | 42.5 | 17,7 | 0,4 | 0,06 |

L'exemple ci-dessus montre une corrélation nette entre la dose de composé n°1 testé et

- la diminution de cellules au stade de la mitose
- l'augmentation de cellules en apoptose.

**Exemple 10 : Influence de l'action du composé n°1 sur les étapes du cycle cellulaire**

L'analyse par cytométrie de flux permet de mettre en évidence un blocage dans une phase particulière du cycle cellulaire après traitement par un composé.

Les cellules HCT-116 sont ensemencées en boîtes 6 puits Nunc. Le composé n°1 à une concentration de 1µg/ml est mis en contact avec les cellules pendant 4 heures, 1 jour, 2 jours et 3 jours avant l'analyse.

L'analyse est effectuée à l'aide d'un test au BrDU : (Dolbeare F. et al, Proc. Natl. Acad. Sci. USA, 1983, 80,

p. 5573- 5577). Les cellules sont traitées par le BrDU à 30 µM pendant 30 minutes, puis trypsinées. Après fixation des cellules dans du paraformaldéhyde à 1 % pendant 16 heures, celles-ci sont digérées dans de la pepsine chlorhydrique, puis rincées dans du PBS 1X. Un marquage immunologique est réalisé avec un anticorps primaire anti-BrDU (Becton Dickinson) et un anticorps secondaire GAM-FITC (Coulter). L'ADN est ensuite marqué avec de l'iodure de propidium dans du PBS contenant 1 mg/ml de RNAse bouillie. Cette méthode permet de comptabiliser les cellules en phase G1, S et G2M.

| Etude par cytométrie de flux des modifications du cycle cellulaire induites par le composé n° 1 sur la lignée HCT-116 | | | | |
|---|---|---|---|---|
| HCT-116 | Temps de contact | Cellules en phase G1 (%) | Cellules en phase S (%) | Cellules en phase G2-M (%) |
| Cellules non traitées | 4 h | 70.8 | 16.7 | 10.8 |
| | 24 h | 65.6 | 19.3 | 13.8 |
| | 48 h | 65.0 | 19.9 | 13.5 |
| | 72 h | 65.8 | 20.1 | 12.8 |
| Cellules traitées (1 µg/mL du composé n°1) | 4 h | 62.2 | 20.1 | 16.4 |
| | 24 h | 26.8 | 39.5 | 31.4 |
| | 48 h | 7.9 | 30.9 | 58.9 |
| | 72 h | 7.8 | 5.1 | 82.4 |

Dans les cellules non traitées, la proportion de cellules en transition G2/M est de l'ordre de 10 à 14 % alors que dans les cellules préalablement mises en contact avec le composé n°1, ce taux augmente jusqu'à atteindre plus de 80 % au bout de 72 heures. Ainsi, dans cette expérience, presque toutes les cellules sont stoppées en phase G2/M par l'action du composé n°1.

## Revendications

1. Composé de formule (I) :

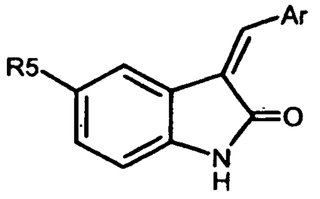

dans laquelle R5 est choisi parmi les groupements 3-pyridinyle, 5-pyrimidinyle, -CONH-alkyle en C1-C4, -NHCO-alkyle en C1-C4, $-CO_2R$ où R peut être hydrogène ou alkyle en C1-C4, $-SO_2NH_2$, $-NO_2$, $-CF_3$,

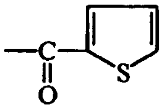

dans laquelle Ar est choisi parmi les groupements 5-imidazolyle, 2-pyrrolyle éventuellement substitués par un radical alkyle en C1-C4, 2-furyle, ou 2-thiazolyle, sous la forme E, Z ou un mélange des deux formes d'isomères.

2. Composé selon la revendication 1 **caractérisé par le fait que** R5 est un groupement 3-pyridinyle ou -CONH-méthyle ou -NHCO-méthyle.

3. Composé selon la revendication 1 ou 2 **caractérisé par le fait que** Ar est un groupement 5-imidazolyle ou un 5-(4-methyl-imidazolyle) ou un 2-pyrrolyle.

4. Composé selon l'une quelconque des revendications 1 à 3 **caractérisé par le fait qu'**il est choisi parmi :

   3-(imidazol-4-méthylydènyl)-5-(pyridin-3-yl)-indolin-2-one,
   3-(pyrrol-2-méthylydènyl)-5-(pyridin-3-yl)-indolin-2-one,
   3-(imidazol-4-méthylydènyl)-5-N-méthylcarboxamido-indolin-2-one,
   3-(imidazol-4-méthylydènyl)-5-acétylamino-indolin-2-one.

5. Procédé de préparation des composés de formule (I) **caractérisé par le fait que** l'on soumet le composé de formule (II) :

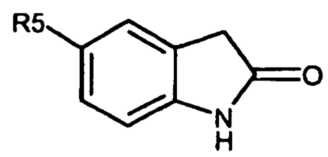

   dans laquelle R5 est un groupement choisi parmi 3-pyridinyle, 5-pyrimidinyle, -CONH-alkyle en C1-C4, -NHCO-alkyle en C1-C4, $-CO_2R$ où R peut être hydrogène ou alkyle en C1-C4, $-SO_2NH_2$, $-NO_2$, $-CF_3$,

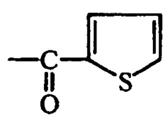

   à une réaction avec un composé de formule (III) :

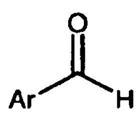

,

   où Ar est choisi parmi les groupements 5-imidazolyle, 2-pyrrolyle éventuellement substitués par un radical alkyle en C1-C4, 2-furyle, ou 2-thiazolyle.

6. Procédé selon la revendication 5, **caractérisé par le fait que** la réaction s'effectue en présence de pipéridine et d'éthanol au reflux.

7. Composé selon l'une quelconque des revendications 1 à 4 pour son application comme médicament.

8. Composition pharmaceutique **caractérisée par le fait qu'**elle comprend dans un milieu pharmaceutiquement acceptable, un composé selon l'une quelconque des revendications 1 à 4.

9. Médicament **caractérisé par le fait qu'**il contient au moins un composé composé selon l'une quelconque des revendications 1 à 4 pour le traitement des maladies associées à une dérégulation d'un complexe enzymatique responsable de la transition G2/M dans le cycle cellulaire.

10. Médicament selon la revendication 9 pour son application thérapeutique dans le traitement des tumeurs primaires et secondaires des cancers.

11. Médicament selon la revendication 9 pour son application dans le traitement du cancer par action inhibitrice sur les kinases dépendantes des cyclines (CDK).

**12.** Médicament selon la revendication 9 pour son application dans le traitement du cancer par action inhibitrice sur le CDK-1.

**13.** Utilisation d'un composé selon l'une quelconque des revendications 1 à 4 pour la préparation d'un médicament destiné au traitement du cancer.

**14.** Utilisation d'un composé selon l'une quelconque des revendications 1 à 4 pour la préparation d'un médicament destiné au traitement du cancer en combinaison avec un traitement par chimiothérapie, radiothérapie ou un traitement anti-angiogénique.

## Patentansprüche

**1.** Verbindung der Formel (I):

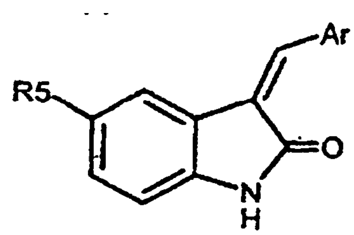

worin R5 ausgewählt ist unter den Gruppen 3-Pyridinyl, 5-Pyrimidinyl, -CONH-(C1-C4)-Alkyl, -NHCO-(C1-C4)-Alkyl, $-CO_2R$, worin R Wasserstoff oder C1-C4-Alkyl sein kann, $-SO_2NH_2$, $-NO_2$, $-CF_3$,

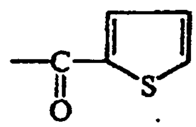

worin Ar ausgewählt ist unter den Gruppen 5-Imidazolyl, 2-Pyrrolyl, die gegebenenfalls mit einem C1-C4-Alkylrest substituiert sind, 2-Furyl oder 2-Thiazolyl, in E,Z-Form oder ein Gemisch der beiden Isomerformen.

**2.** Verbindung gemäß Anspruch 1, **dadurch gekennzeichnet, dass** R5 eine 3-Pyridinyl- oder -CONH-Methyl- oder -NHCO-Methyl-Gruppe ist.

**3.** Verbindung gemäß Anspruch 1 oder 2, **dadurch gekennzeichnet, dass** Ar eine 5-Imidazolyl- oder eine 5-(4-Methyl-imidazolyl)- oder eine 2-Pyrrolyl-Gruppe ist.

**4.** Verbindung gemäß einem der Ansprüche 1 bis 3, **dadurch gekennzeichnet, dass** sie ausgewählt ist unter:

3-(Imidazol-4-methylidenyl)-5-(pyridin-3-yl)-indolin-2-on,
3-(Pyrrol-2-methylidenyl)-5-(pyridin-3-yl)-indolin-2-on,
3-(Imidazol-4-methylidenyl)-5-N-methylcarboxamido-indolin-2-on,
3-(Imidazol-4-methylidenyl)-5-acetylamino-indolin-2-on.

**5.** Verfahren zur Herstellung der Verbindungen der Formel (I), **dadurch gekennzeichnet, dass** man die Verbindung der Formel (II):

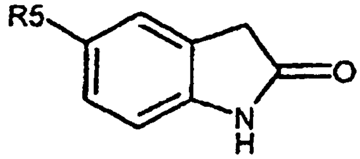

worin R5 eine Gruppe ist, die ausgewählt ist unter 3-Pyridinyl, 5-Pyrimidinyl, -CONH-(C1-C4)-Alkyl, -NHCO-(C1-C4)-Alkyl, $-CO_2R$, worin R Wasserstoff oder C1-C4-Alkyl sein kann, $-SO_2NH_2$, $-NO_2$, $-CF_3$,

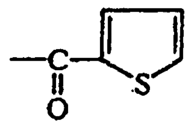

einer Reaktion mit einer Verbindung der Formel (III):

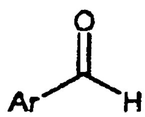

worin Ar ausgewählt ist unter den Gruppen 5-Imidazolyl, 2-Pyrrolyl, die gegebenenfalls mit einem C1-C4-Alkylrest substituiert sind, 2-Furyl oder 2-Thiazolyl, unterzieht.

6. Verfahren gemäß Anspruch 5, **dadurch gekennzeichnet, dass** die Reaktion in Gegenwart von Piperidin und Ethanol am Rückfluss erfolgt.

7. Verbindung gemäß einem der Ansprüche 1 bis 4 für ihre Anwendung als Arzneimittel.

8. Pharmazeutische Zusammensetzung, **dadurch gekennzeichnet, dass** sie in einem pharmazeutisch annehmbaren Medium eine Verbindung gemäß einem der Ansprüche 1 bis 4 umfasst.

9. Arzneimittel, **dadurch gekennzeichnet, dass** es wenigstens eine Verbindung gemäß einem der Ansprüche 1 bis 4 enthält, für die Behandlung der Krankheiten, die mit einer Deregulierung eines Enzymkomplexes, der für den Übergang G2/M im Zellzyklus verantwortlich ist, zusammenhängen.

10. Arzneimittel gemäß Anspruch 9 für seine therapeutische Anwendung bei der Behandlung von primären und sekundären Krebstumoren.

11. Arzneimittel gemäß Anspruch 9 für seine Anwendung bei der Behandlung von Krebs durch inhibierende Wirkung auf die Cyklin-abhängigen Kinasen (CDK).

12. Arzneimittel gemäß Anspruch 9 für seine Anwendung bei der Behandlung von Krebs durch inhibierende Wirkung auf CDK-1.

13. Verwendung einer Verbindung gemäß einem der Ansprüche 1 bis 4 für die Herstellung eines Arzneimittels, das für die Behandlung von Krebs bestimmt ist.

14. Verwendung einer Verbindung gemäß einem der Ansprüche 1 bis 4 für die Herstellung eines Arzneimittels, das für die Behandlung von Krebs in Kombination mit einer Behandlung durch Chemotherapie, Strahlentherapie oder einer antiangiogenetischen Behandlung bestimmt ist.

## Claims

**1.** Compound of formula (I):

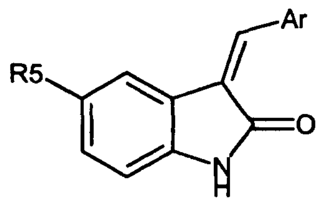

in which R5 is chosen from the following groups: 3-pyridyl, 5-pyrimidinyl, -CONH-($C_1$-$C_4$ alkyl), -NHCO-($C_1$-$C_4$ alkyl), $-CO_2R$ where R can be hydrogen or $C_1$-$C_4$ alkyl, $-SO_2NH_2$, $-NO_2$, $-CF_3$ or

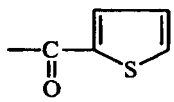

in which Ar is chosen from the following groups: 5-imidazolyl, 2-pyrrolyl, which are optionally substituted by a $C_1$-$C_4$ alkyl radical, 2-furyl or 2-thiazolyl, in the E or Z form or a mixture of the two forms of isomers.

**2.** Compound according to Claim 1, **characterized in that** R5 is a 3-pyridyl or -CONH-methyl or -NHCO-methyl group.

**3.** Compound according to Claim 1 or 2, **characterized in that** Ar is a 5-imidazolyl or a 5-(4-methylimidazolyl) or a 2-pyrrolyl group.

**4.** Compound according to any one of Claims 1 to 3, **characterized in that** it is chosen from:

3-(imidazol-4-methylidenyl)-5-(pyrid-3-yl)indolin-2-one,
3-(pyrrol-2-methylidenyl)-5-(pyrid-3-yl)indolin-2-one,
3-(imidazol-4-methylidenyl)-5-(N-methylcarboxamido)indolin-2-one,
3-(imidazol-4-methylidenyl)-5-(acetylamino)indolin-2-one.

**5.** Process for the preparation of the compounds of formula (I), **characterized in that** the compound of formula (II):

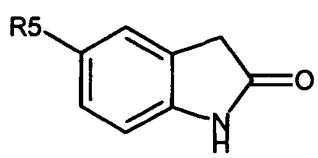

in which R5 is a group chosen from the following groups: 3-pyridyl, 5-pyrimidinyl, -CONH-($C_1$-$C_4$ alkyl), -NH-CO-($C_1$-$C_4$ alkyl), $-CO_2R$ where R can be hydrogen or $C_1$-$C_4$ alkyl, $-SO_2NH_2$, $-NO_2$, $-CF_3$ or

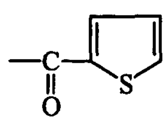

,

is reacted with a compound of formula (III):

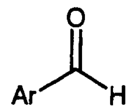

where Ar is chosen from the following groups: 5-imidazolyl, 2-pyrrolyl, which are optionally substituted by a $C_1$-$C_4$ alkyl radical, 2-furyl or 2-thiazolyl.

**6.** Process according to Claim 5, **characterized in that** the reaction is carried out in the presence of piperidine and of ethanol at reflux.

**7.** Compound according to any one of Claims 1 to 4, for its application as medicament.

**8.** Pharmaceutical composition, **characterized in that** it comprises, in a pharmaceutically acceptable medium, a compound according to any one of Claims 1 to 4.

**9.** Medicament, **characterized in that** it comprises at least one compound according to any one of Claims 1 to 4 for the treatment of diseases associated with deregulation of an enzymatic complex responsible for the G2/M transition in the cell cycle.

**10.** Medicament according to Claim 9, for its therapeutic application in the treatment of primary and secondary tumours of cancers.

**11.** Medicament according to Claim 9, for its application in the treatment of cancer by an inhibitory action on cyclin-dependent kinases (CDKs).

**12.** Medicament according to Claim 9, for its application in the treatment of cancer by an inhibitory action on CDK-1.

**13.** Use of a compound according to any one of Claims 1 to 4, for the preparation of a medicament intended for the treatment of cancer.

**14.** Use of a compound according to any one of Claims 1 to 4, for the preparation of a medicament intended for the treatment of cancer in combination with a treatment by chemotherapy or radiotherapy or an antiangiogenic treatment.

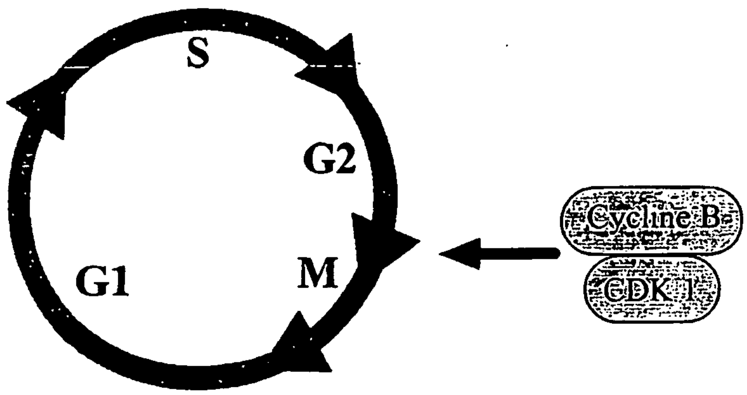


Figure 1